# EUROPEAN PATENT APPLICATION

(11) **EP 3 824 850 A1**
(43) Date of publication of application: **26.05.2021**
(21) Application number: 19838846.4
(22) Date of filing: 22.07.2019
(51) Int. Cl.: A61F 2/60

(54) **SOLE FOR ARTIFICIAL FEET**

(30) Priority: 20.07.2018 JP 2018137196
(71) Applicant: Bridgestone Corporation, Chuo-Ku Tokyo 104-8340 (JP)
(72) Inventor: ITOI Dyta, Tokyo 104-8340 (JP); SAHASHI Kohei, Tokyo 104-8340 (JP)
(74) Representative: Oxley, Robin John George
(86) International application number: PCT/JP2019/028718
(87) International publication number: WO 2020/017663

(57) **Abstract**

A sole for a prosthetic leg is a sole which is attached to a ground contact region of the prosthetic leg with a road surface and includes a through hole at least on a partial region.

## Description

### TECHNICAL FIELD

The present disclosure relates to a sole attached to a ground contact region of a prosthetic leg.

### BACKGROUND

Conventionally, a prosthetic leg for athletics (hereinafter, referred to as an athletic prosthetic leg or simply referred to as a prosthetic leg) having a leaf-spring-like leg portion which has a curved portion and extends to a side of a toe and in which a ground contact region extends from the toe to a side of the curved portion in an arc has been well-known. To such an athletic prosthetic leg having the leaf-spring-like leg portion, generally, a sole which abuts a road surface is attached to a bottom surface of the ground contact region.

For example, Patent Literature 1 (PLT 1) illustrates a sole attached to a lower surface of a curved leaf-spring-like athletic prosthetic leg in accordance with sporting events. More specifically, Patent Literature 1 discloses a sole to which a spike is attached at a lower surface of the sole contacting a road surface or a sole provided with a number of outsole portions each having a hexagonal contact patch.

### CITATION LIST

### Patent Literature

PTL 1: Japanese Patent Laid-Open No. 2016-150189

### SUMMARY

### (Technical Problem)

However, in the sole illustrated in Patent Literature 1, weight saving of the sole of the prosthetic leg is not at all considered. For example, in the case of athletics with an athlete wearing the prosthetic leg, a burden to be placed on the athlete can be smaller as the weight of the sole of the prosthetic leg becomes lighter.

An object of the present disclosure is to provide a lightweight sole for an athletic prosthetic leg.

### (Solution to Problem)

The gist of the present disclosure is as follows.
(1) A sole for a prosthetic leg, the sole being configured to be attached to a ground contact region of the prosthetic leg with a road surface, the sole including a through hole at least on a partial region.
(2) A sole for a prosthetic leg, the sole being configured to be attached to a ground contact region of the prosthetic leg with a road surface, the sole being divided into a plurality of sole portions.

### (Advantageous Effect)

According to the sole for a prosthetic leg according to the present disclosure, a lightweight sole for a prosthetic leg can be provided.

### BRIEF DESCRIPTION OF THE DRAWINGS

In the accompanying drawings:
FIG. 1 is a side view of an athletic prosthetic leg to which a sole according to a first embodiment of the present disclosure is attached;
FIG. 2A is a side view for explaining in stages movement of a leg portion and a ground contact form in a case where the athletic prosthetic leg is worn and a wearer executes straight running;
FIG. 2B is a side view for explaining in stages the movement of the leg portion and the ground contact form in a case where the athletic prosthetic leg is worn and the wearer executes straight running;
FIG. 2C is a side view for explaining in stages the movement of the leg portion and the ground contact form in a case where the athletic prosthetic leg is worn and the wearer executes straight running;
FIG. 2D is a side view for explaining in stages the movement of the leg portion and the ground contact form in a case where the athletic prosthetic leg is worn and the wearer executes straight running;
FIG. 3 is a drawing for explaining each region of a bottom surface of the sole;
FIG. 4 is a drawing illustrating an example of a pattern of the bottom surface of the sole;
FIG. 5A is a perspective view illustrating the sole and a tab for sticking before the sole is attached to a ground contact portion;
FIG. 5B is a drawing for explaining the thickness at a portion adjacent to a border between a toe-side tab for sticking and the sole;
FIG. 5C is an enlarged cross-sectional view for illustrating a portion adjacent to and including a border between a curved-portion-side tab for sticking and the sole;
FIG. 6 is a drawing illustrating an example of a pattern of the bottom surface of the sole according to a second embodiment of the present disclosure;
FIG. 7A is a schematic drawing illustrating the sole and a retaining film before the sole is attached to the ground contact portion;
FIG. 7B is a schematic drawing illustrating a state that the sole illustrated in FIG. 7A is adhered to the ground contact portion; and
FIG. 8 is a schematic drawing illustrating an example when a cushion member is interposed between the sole and a ground contact region.

### DETAILED DESCRIPTION

Hereinafter, with reference to the drawings, a sole for an athletic prosthetic leg according to the present disclosure (hereinafter, it is also referred to as a sole) will be explained in detail with illustration of embodiments thereof.

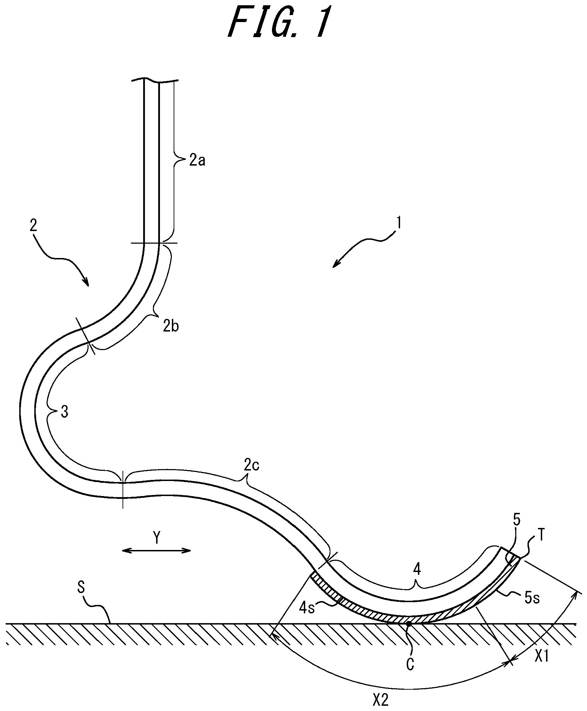

FIG. 1 is a side view of an athletic prosthetic leg 1 to which a sole 5 according to a first embodiment of the present disclosure is attached. The athletic prosthetic leg 1 has a leaf-spring-like leg portion 2, and the sole 5 is attached to a ground contact region at its tip side. Additionally, although not illustrated, a base end portion of the leg portion 2 is connected to a socket via an adapter and the socket houses a stump of a wearer's leg, whereby the wearer can wear the prosthetic leg. The adapter and the socket which correspond to the position of the stump of the leg, such as an above-knee prosthesis and a below-knee prosthesis, are used. FIG. 1 illustrates the leg portion 2 and the sole 5 in a standing state of the wearer who wears the athletic prosthetic leg 1.

Hereinafter, in this embodiment, in a height direction of the athletic prosthetic leg, a side where the leg portion 2 is connected to the adapter is referred to as a connection side, and a side where the leg portion 2 contacts a road surface S is referred to as a ground contact side. Also, a toe T of the athletic prosthetic leg 1 refers to a point at the forefront as a termination of the leg portion 2 extending from the connection side and a point at the forefront of the sole 5 attached to the athletic prosthetic leg 1 to constitute the same plane as the toe T of the athletic prosthetic leg 1. Further, a direction extending from the toe T in parallel with the road surface S is referred to as a leg portion front-rear direction Y. Further, a widthwise direction of the leg portion 2 and the sole 5 in a state of being attached to the leg portion 2 of the athletic prosthetic leg is referred to as a width direction W.

In this embodiment, the leg portion 2 of the athletic prosthetic leg 1 has a plate-like extending shape from the connection side to the side of the toe T via at least one curved portion, in the illustrated example, one curved portion 3. In FIG. 1, the leg portion 2 is constituted by, in the order from the connecting side to the ground contact side, a straight portion 2a, a curved portion 2b which is convex to the side of the toe T, the curved portion 3 which is convex to a rear side in the leg portion front-rear direction Y, a curved portion 2c which is concave to the ground contact side and a ground contact portion 4 which is convex to the ground contact side to extend to the side of the toe T in an arc.

Additionally, although the material of the leg portion 2 is not limited, from a viewpoint of strength and weight saving, fiber reinforced plastic etc. is preferably used.

The ground contact portion 4 includes a ground contact region 4s extending from the toe T to the side of the curved portion 3 in an arc at the ground contact side, and the sole 5 is attached to the ground contact region 4s. The ground contact region 4s refers to the entire region abutting the road surface S when the wearer who wears the athletic prosthetic leg 1 executes running movement, and in a state that sole 5 is attached, the ground contact region 4s abuts the road surface S via the sole 5.

The sole 5 has a shape conforming to an extending shape of the ground contact region 4s. Also, the ground contact side of the sole 5 is a bottom surface 5s. As illustrated in FIG. 1, the bottom surface 5s has a shape in which an arc X1 and an arc X2 are continued from the toe T side to the curved portion 3 side. While the arc X1 and the arc X2 have a different radius of curvature to each other in this embodiment, they may include the same radius of curvature.

Also, the bottom surface 5s has different properties at one side and the other side, which are defined by a border as a line extending in the width direction W through a point C as a contact point with the road surface S in a side view in a standing state of the wearer when the athletic prosthetic leg 1 with the sole 5 attached is worn. The point C is a point which firstly contacts the road surface S in arriving at standing. In other words, the standing state refers to a state that the wearer firstly contacts the road surface S by lowering the athletic prosthetic leg 1 to the road surface S from a state that the wearer supports his body by a healthy leg wearing no prosthetic leg when a prosthetic leg is used for only one leg, or the wearer supports his body by one prosthetic leg when prosthetic legs are used for both legs. Additionally, the point C is determined depending on the shape or an attachment aspect etc. of the prosthetic leg. In other words, the inventors newly conceived that the border for separating functions of the bottom surface 5s from a finding related to a ground contact form obtained from an experiment which will be described later uses the point C which is the contact point with the road surface S in the wearer's standing state as a standard.

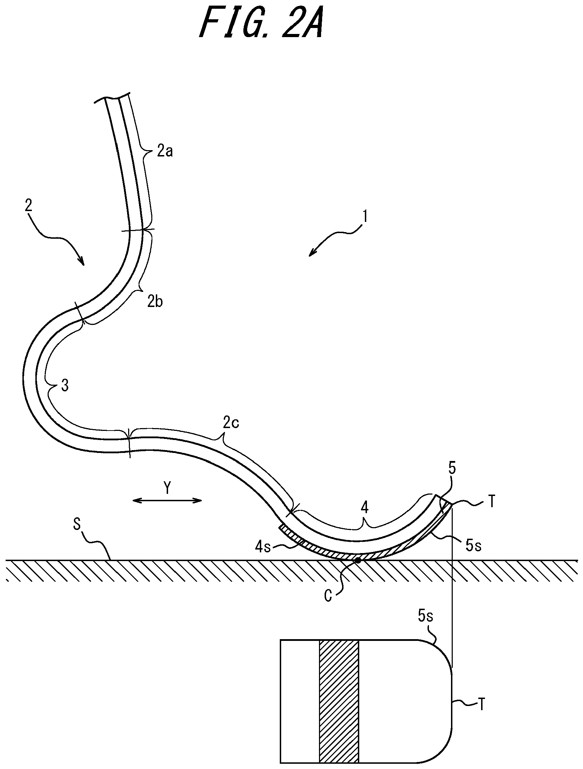

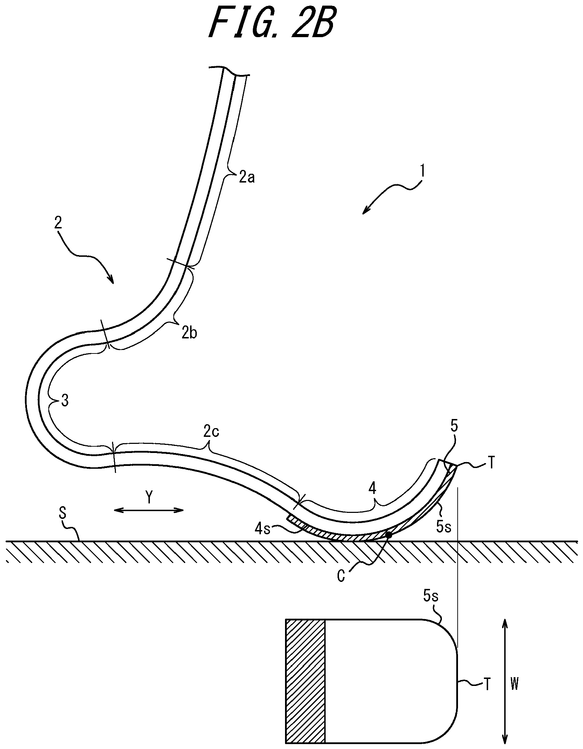

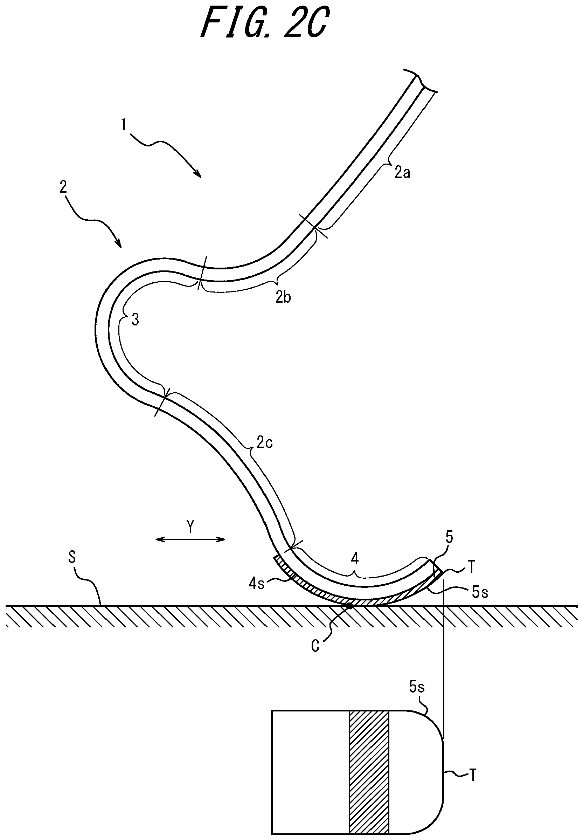

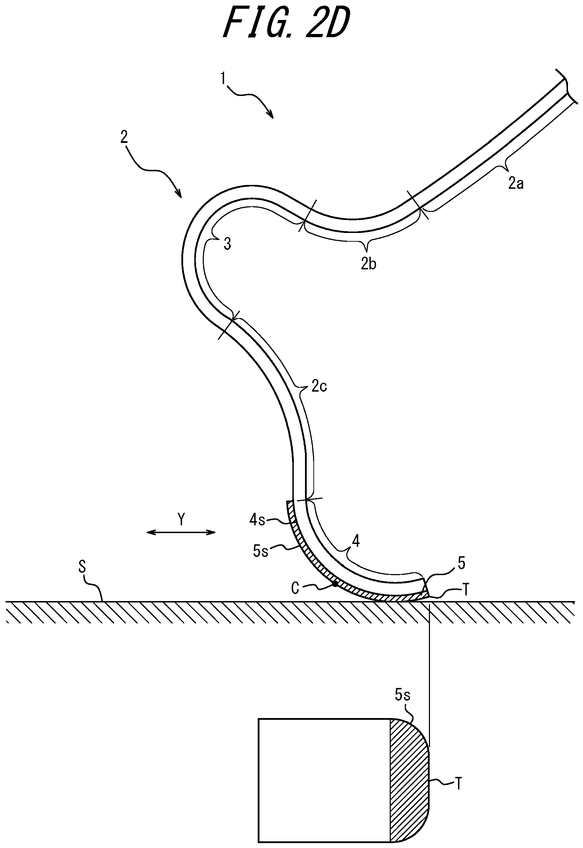

Here, an experiment result of the ground contact form of the bottom surface 5s as described above will be explained using FIGS. 2A, 2B, 2C and 2D. FIGS. 2A, 2B, 2C and 2D are drawings for explaining in stages movement of the leg portion 2 and the ground contact form of the bottom surface 5s when the wearer who wears the athletic prosthetic leg 1 having the above configuration executes straight running. In each drawing, an upper portion is a side view of the leg portion 2 and the sole 5, and a lower portion illustrates a transition of the ground contact form of the bottom surface 5s when the wearer who wears the athletic prosthetic leg 1 executes straight running.

FIG. 2A illustrates a state that the wearer lowers the raised athletic prosthetic leg 1 to the road surface S and the entire weight is loaded on the athletic prosthetic leg 1. As illustrated in the lower portion of the drawing, in the bottom surface 5s, a region at the side of the curved portion 3 from the point C contacts the ground.

FIG. 2B illustrates a state that the wearer stamps on the ground, from the state of FIG. 2A, while the entire weight remains to be loaded on the athletic prosthetic leg 1. In a case of running of a healthy person, such a stamping form is generally applied that firstly a heel side of a shoe sole contacts to the ground and the ground contact is sequentially executed to a toe side, while in the athletic prosthetic leg 1, the ground contact region is moved to the side of the curved portion 3 from a region which firstly contacts the ground.

FIG. 2C illustrates a state that the wearer starts a kick-out movement of the athletic prosthetic leg 1 by swinging an opposite leg from the leg wearing the athletic prosthetic leg 1 forward. Entering into this kick-out movement, in the athletic prosthetic leg 1, a region at the side of the toe T from the point C of the bottom surface 5s contacts the ground.

FIG. 2D illustrates a state that the wearer is in a final stage of kicking out the athletic prosthetic leg 1 just before separating from the road surface S. To kick out from the toe T of the bottom surface 5s, the athletic prosthetic leg contacts the ground further at the side of the toe T than in FIG. 2C. At this time, in accordance with ground contact pressure applied from the ground contact region of the prosthetic leg to the road surface S, a reaction force of a plate spring acts, and due to this reaction force, a propulsive force is generated.

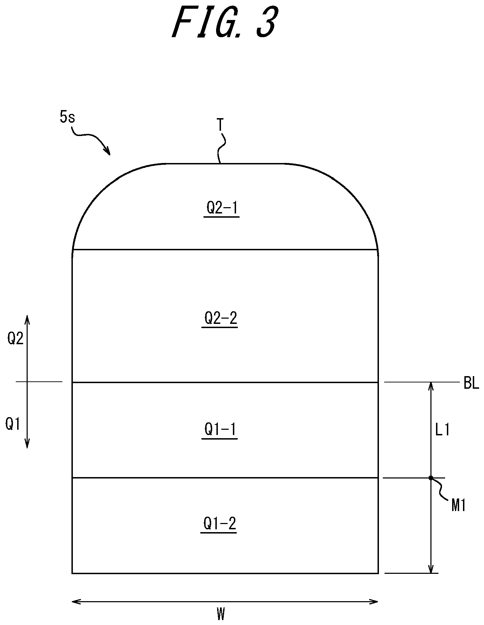

Based on the experimental result illustrated in FIGS. 2A, 2B, 2C and 2D, as illustrated in FIG. 3, firstly, the bottom surface 5s is divided into a heel side region Q1 and a toe side region Q2 with a border of the point C.

The heel side region Q1 is a region at the side of the curved portion 3 defined by a border as a line BL extending in the width direction W of the leg portion 2 through the point C seen from the bottom surface 5s illustrated in FIG. 3. As illustrated in FIGS. 2A and 2B, the heel side region Q1 is a region where the wearer firstly contacts the ground and executes a step movement in a state that the entire weight is loaded to the athletic prosthetic leg 1. Consequently, it is vital that the heel side region Q1 fully grips the road surface S such that the entire body is balanced even when the entire weight of the wearer is loaded on the athletic prosthetic leg 1. Thus, to prevent slip due to a water film interposed between the bottom surface 5s and the road surface S, drainage performance of the heel side region Q1 needs to be higher than a portion other than the heel side region Q1, that is, the toe side region Q2.

In other words, since the heel side region Q1 has a higher drainage performance compared with the portion other than the heel side region Q1, the sole 5 of the athletic prosthetic leg 1 prevents slip due to the water film and achieves a high anti-slip property.

On the other hand, the toe side region Q2 is a region at the side of the toe T defined by the border as the line BL extending in the width direction W of the leg portion 2 through the point C seen from the bottom surface 5s illustrated in FIG. 3. The toe side region Q2 is a region where the wearer swings an opposite leg from a leg wearing the athletic prosthetic leg 1 forward to execute the kick-out movement of the athletic prosthetic leg 1. The toe side region Q2 sequentially contacts the ground toward the toe T, and the wearer presses the road surface S by the bottom surface 5s to slidingly contact the ground, so that the toe side region Q2 is a region which easily develops abrasion in particular. Thus, wear resistance performance of the toe side region Q2 needs to be higher than that of the heel side region Q1.

In other words, with the toe side region Q2 having a higher wear resistance performance than the heel side region Q1, early abrasion of the toe side region Q2 is avoided, and as a result, the entire surface of the sole 5 of the athletic prosthetic leg 1 is gently worn and a long service life of the sole 5 can be achieved.

Also, it is preferable that each of the heel side region Q1 and the toe side region Q2 is further divided as illustrated in FIG. 3 based on the ground contact form illustrated in FIGS. 2A to 2D such that each portion has property corresponding to the ground contact form.

In the toe side region Q2 illustrated in FIG. 3, a portion Q2-1 corresponds to the arc X1 which continues from the toe T with a constant radius of curvature in FIG. 1. The portion Q2-1 contacts the ground at the end when the wearer who wears the athletic prosthetic leg 1 executes the kick-out movement, so that severer abrasion has been inclined to occur. Thus, the portion Q2-1 needs to have an especially high wear resistance performance. In other words, in the toe side region Q2, the portion Q2-1 has a higher wear resistance performance than a remaining portion Q2-2, so that the sole 5 is protected from severe abrasion and the long service life of the sole 5 can be achieved.

In the heel side region Q1, a portion Q1-1 at the side of the toe T from a center M1 of a maximum length L1 along the leg portion front-rear direction Y is a region which firstly contacts the ground, so that prevention of slip is especially necessary such that the wearer achieves a balance of his body. Thus, the portion Q1-1 preferably has a further higher drainage performance than a remaining portion Q1-2 in the heel side region Q1 such that slip is more surely prevented and a further stable running is achieved.

Also, the portion Q1-2 is a portion at the side of the curved portion 3 from the center M1 of the maximum length L1. As illustrated in FIG. 2B, in the heel side region Q1, the ground contact portion is shifted to the side of the curved portion 3 from the portion Q1-1 which firstly contacts the ground, that is, the portion Q1-2 at the opposite side from a direction that the wearer advances. At the time of ground contact of the portion Q1-2, movement of an upper body in which the wearer tries to move forward and movement of the ground contact portion are temporarily opposite, thus, a high propulsive force is needed for the kick-out movement at the latter half of the ground contact form. Consequently, firstly, it is vital that the portion Q1-2 has a higher rigidity than the portion Q1-1. Since the portion Q1-2 has a higher rigidity than the portion Q1-1, the step movement is smoothly continued to the kick-out movement, and a high propulsive force can be achieved.

Especially, in a case where the bottom surface 5s includes a pattern constituted by a plurality of recesses and protrusions, the portion Q1-2 preferably has a larger edge component in the width direction W of the leg portion 2 than the portion Q1-1. Also, a negative ratio of the portion Q1-2 is preferably smaller than that of the portion Q1-1. Here, the negative ratio refers to a percentage in an area of a recessed portion to the road surface S in a planar view in a total area of the bottom surface 5s in a planar view. With this configuration, a high propulsive force can be exerted in running.

Also, to exert the propulsive force effectively, the portion Q1-2 preferably has a smaller edge component in the width direction W of the leg portion 2 than the toe side region Q2. Further, a negative ratio of the portion Q1-2 is preferably larger than that of the toe side region Q2. With this configuration, the portion Q1-2 can exert a high propulsive force when the wearer executes the kick-out movement.

Specific means to achieve the above-described properties to be applied to each portion of the bottom surface 5s includes, for example, designing a pattern constituted by recesses and protrusions by grooves and the like formed on the bottom surface 5s, designing the surface property of the bottom surface 5s, designing the cross-sectional shape of the sole 5 and designing the material of the sole 5.

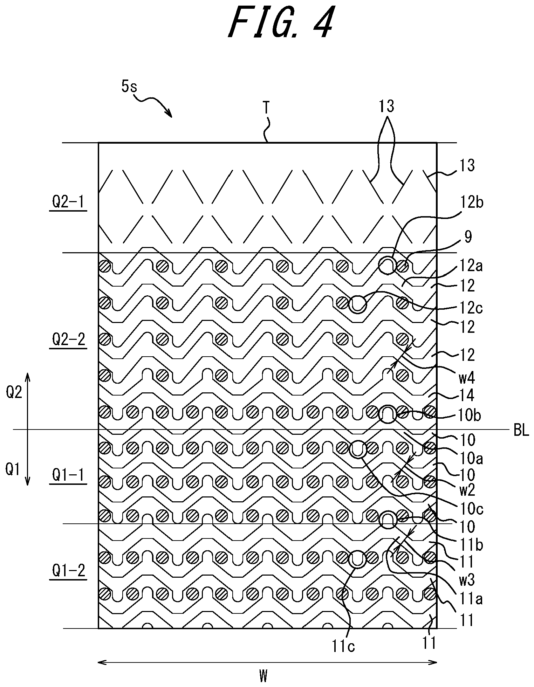

Here, a case where each function is applied by design of the pattern constituted by recesses and protrusions of the bottom surface 5s will be explained. FIG. 4 is a drawing illustrating an example of a pattern of the bottom surface 5s of the sole 5 in the athletic prosthetic leg 1.

In the pattern illustrated in FIG. 4, a plurality of land portions 10 and a plurality of land portions 11 which are defined by a plurality of grooves extending in the width direction W are arranged in the heel side region Q1. The land portions 10 are arranged to the side of the toe T from the land portions 11. The land portions 10 are shaped to include a width direction extending portion 10a extending in the width direction W to be substantially zigzag-shaped, a toe side protruding portion 10b extending to the side of the toe T from a bent portion bending to be convex to the side of the toe T and a curved portion side protruding portion 10c extending to the side of the curved portion 3 from a bent portion bending to be convex to the side of the curved portion 3. The land portions 11 are shaped to include a width direction extending portion 11a, a toe side protruding portion 11b and a curved portion side protruding portion 11c. The width direction extending portions 10a and 11a are zig-zag shaped, thereby fully ensuring the edge component. Further, by forming the toe side protruding portions 10b and 11b as well as the curved portion side protruding portions 10c and 11c, the edge component is further increased, and the water film interposed between the bottom surface 5s and the road surface S can be efficiently cut on both sides in a ground contact region extending direction, thereby achieving a high drainage performance.

Also, in FIG. 4, a plurality of land portions 12 which are defined by a plurality of grooves extending in the width direction W are arranged in the toe side region Q2. The land portions 12 are shaped to include a width direction extending portion 12a extending in the width direction W to be substantially zig-zag shaped, a toe side protruding portion 12b extending to be convex in a direction that the width direction extending portion 12a extends from a bent portion bending to be convex to the side of the toe T and a curved portion side protruding portion 12c extending to be convex in a direction that the width direction extending portion 12a extends from a bent portion bending to be convex to the side of the curved portion 3. Further, a plurality of linear grooves 13 intermittently extending along the zig-zag shape extending in the width direction W are formed in the toe side region Q2. The land portions 12 are arranged to the side of the curved portion 3 from the linear grooves 13 and the linear grooves 13 are formed to the side of the toe T from the land portions 12. Additionally, as illustrated in the drawing, a land portion 14 having the same shape as the land portions 11 may be formed in the toe side region Q2.

In FIG. 4, a land portion width w2 of the width direction extending portion 10a of the land portions 10 is larger than a land portion width w3 of the width direction extending portion 11a of the land portions 11. Also, a land portion width w4 of the width direction extending portion 12a of the land portions 12 is larger than the land portion widths w2 and w3. As understood from FIG. 4, each of the land portions 10, 11, 12 and 14 is defined by the groove portions.

In this configuration, in the heel side region Q1, a percentage in an area of a groove portion which is concave to the road surface S in a planar view in a total area of the bottom surface 5s in a planar view, that is, a negative ratio is larger than that in the toe side region Q2. Thus, in the heel side region Q1, more water can be taken in a recessed groove and can be discharged. Thus, the heel side region Q1 has a higher drainage performance than the toe side region Q2.

On the other hand, the toe side region Q2 has a higher wear resistance performance than the heel side region Q1. The reason is that the toe side region Q2 has a smaller negative ratio than the heel side region Q1 to maintain a high rigidity.

Additionally, in the toe side region Q2, the linear groove 13 is formed in the portion Q2-1. With this configuration, the ground contact portion Q2-1 has a larger rigidity than the remaining portion Q2-2 in the toe side region Q2 to include a further higher wear resistance performance.

Also, in FIG. 4, in the heel side region Q1, the negative ratio of the portion Q1-1 is larger than that of the portion Q1-2, thereby more water can be taken in the grooves and can be discharged. In other words, the portion Q1-1 has a further higher drainage performance than the portion Q1-2.

Further, in the heel side region Q1, the land portions 11 are arranged in the portion Q1-2. Moreover, as described before, the land portion width w3 of the land portions 11 is larger than the land portion width w2 of the land portions 10. Thus, the portion Q1-2 has a larger land portion rigidity than the portion Q1-1. Further, the portion Q1-2 has a larger edge component in the width direction W than the portion Q1-1. Also, as described before, the negative ratio of the portion Q1-2 is smaller than that of the portion Q1-1.

Also, the portion Q1-2 has a smaller edge component in the width direction W than the toe side region Q2 and further, has a larger negative ratio.

In this embodiment, the sole 5 has a through hole 9 at least on a partial region. Consequently, in an attached state to the ground contact region 4s of the athletic prosthetic leg 1, at least a part of the ground contact region 4s is exposed. In this embodiment, the sole 5 is attached to the ground contact region 4s of the athletic prosthetic leg 1.

For example, as illustrated in FIG. 4, the sole 5 has the through hole 9 at a partial region thereof. In FIG. 4, the through hole 9 of the sole 5 is illustrated by a region with oblique lines. In other words, in the region with oblique lines in FIG. 4, at a side of the bottom surface 5s of the sole 5, the ground contact region 4s of the athletic prosthetic leg 1 is exposed.

In an example illustrated in FIG. 4, the sole 5 includes the through hole 9 at regions other than the land portions 10, 11, 12 and 14 in the portions Q2-2, Q1-1 and Q1-2, that is, at the groove portions. In this way, since the sole 5 has the through hole 9, weight saving of the sole 5 can be attained compared to a case where the through hole 9 is not included. Also, the sole 5 has the through hole 9 at the regions other than the land portions 10, 11, 12 and 14, so that weight saving can be attained while maintaining an effect caused by the land portions 10, 11, 12 and 14, for example, drainage performance. Additionally, a pattern of the through hole 9 in the sole 5 is not limited to that illustrated in FIG. 4. The sole 5 may include the through hole 9 with an arbitrary pattern in the regions other than the land portions 10, 11, 12 and 14 in the portions Q2-2, Q1-1 and Q1-2.

In the example illustrated in FIG. 4, the portion Q2-1 of the sole 5 does not include any through hole 9. In this case, wear resistance performance in the portion Q2-1 of the sole 5 is maintained. The sole 5 may include the through hole 9 in the portion Q2-1. In this case, further weight saving of the sole 5 can be achieved.

Also, in the athletic prosthetic leg 1 according to this embodiment, the sole 5 may be attached to the ground contact region 4s via an adhesive. However, attachment means is not limited to the adhesive, and attachment may be executed using fasteners such as a belt.

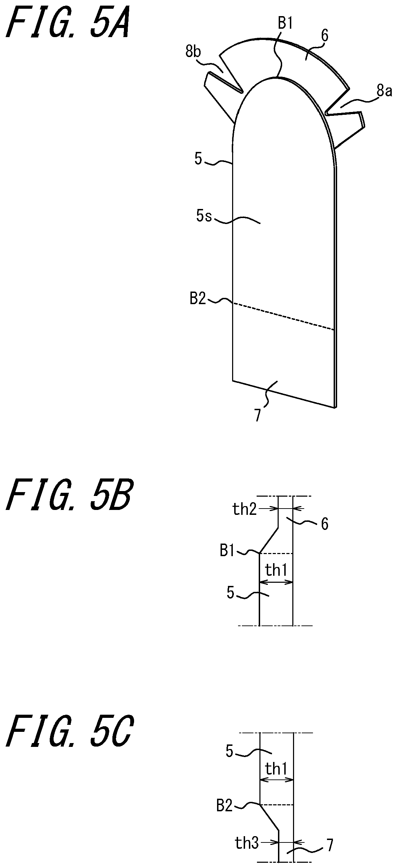

Here, an example of attachment means of the sole 5 according to this embodiment will be explained with reference to FIGS. 5A, 5B and 5C. FIG. 5A is a perspective view illustrating the sole 5 and a tab for sticking before the sole 5 is attached to the ground contact portion 4. Note that, in FIG. 5A, a pattern of the bottom surface 5s and the through holes 9 are not illustrated. As illustrated, a toe-side tab for sticking 6 and a curved-portion-side tab for sticking 7 are integrally connected to the sole 5. The toe-side tab for sticking 6 is fan-shaped and connected along an end edge at the side of the toe T of the sole 5, and moreover, is divided by two cuts 8a, 8b. The curved-portion-side tab for sticking 7 is connected to an end edge at the side of the curved portion 3 of the sole 5. FIG. 5B is a drawing for explaining the thickness at a portion adjacent to and including a border between the toe-side tab for sticking 6 and the sole 5. In addition, FIG. 5C is a drawing for explaining the thickness at a portion adjacent to and including a border between the curved-portion-side tab for sticking 7 and the sole 5. As illustrated in FIG. 5B, the toe-side tab for sticking 6 extends with a constant thickness th2 which is thinner than a thickness th1 of the sole 5, and the thickness gradually increases toward a border B1 with the sole 5. Also, the curved-portion-side tab for sticking 7 extends with a thickness th3 which is thinner than the thickness th1 of the sole 5, and the thickness gradually increases toward a border B2 with the sole 5. With this configuration, when the sole is attached to the ground contact portion 4, close attachment can be executed without any deflection or gap between the sole 5 and the ground contact portion 4. For example, assuming that the thickness th1 of the sole 5 is 2.25 to 3.0 mm, the thickness th2 of the toe-side tab for sticking 6 and the thickness th3 of the curved-portion-side tab for sticking 7 may be 1.5 to 2.0 mm. Additionally, it should be understood that the tab for sticking explained with reference to FIGS. 5A, 5B and 5C does not constitute a part of the sole 5 according to the first embodiment, but is used for attachment of the sole 5.

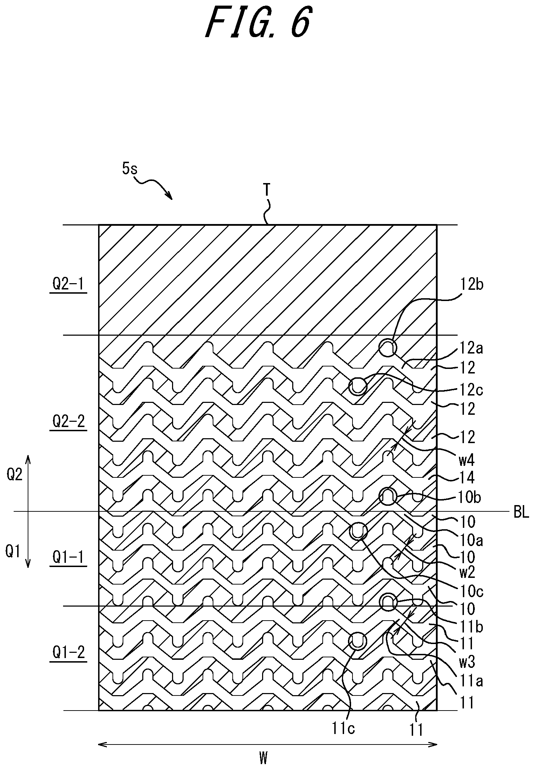

FIG. 6 is a drawing illustrating an example of the pattern of the bottom surface of the sole 5 according to a second embodiment of the present disclosure. Hereinafter, regarding the second embodiment, an explanation of the content which is common to the first embodiment is appropriately omitted, and mainly a different point from the first embodiment will be explained.

In this embodiment, the sole 5 is divided into a plurality of sole portions. In this embodiment, the sole 5 is constituted by the land portions 10, 11, 12 and 14 each of which is independent (separated). In other words, in this embodiment, the plurality of sole portions constituting the sole 5 are the land portions 10, 11, 12 and 14 each of which is independent (separated). In the attached state to the ground contact region 4s of the athletic prosthetic leg 1, regions other than regions to which the plurality of sole portions are attached are exposed at a side of the bottom surface 5s of the sole 5. Consequently, unlike the first embodiment, in this embodiment, the entire region excluding the land portions 10, 11, 12 and 14 is exposed at the side of the bottom surface of the sole 5. In an example illustrated in FIG. 6, in this embodiment, regions with oblique lines are exposed. In other words, in this embodiment, the ground contact region 4s of the athletic prosthetic leg 1 is covered only at regions of the land portions 10, 11, 12 and 14.

As understood from the above explanation and FIG. 6, in this embodiment, each of the land portions 10, 11, 12 and 14 is not connected to each other, unlike the first embodiment.

Also in a case where the entire region excluding the land portions 10, 11, 12 and 14 is exposed at the side of bottom surface of the sole 5 as in this embodiment, weight saving of the sole 5 can be achieved. Also, since the sole 5 is constituted by the land portions 10, 11, 12 and 14, weight saving can be achieved while maintaining an effect caused by the land portions 10, 11, 12 and 14, for example, drainage performance.

In this embodiment, for example, the portion Q2-1 of the sole 5 is not necessarily exposed. For example, in a region of the portion Q2-1, the ground contact region 4s may be covered by the sole 5. Due to this, wear resistance performance at the portion Q2-1 of the sole 5 can be maintained.

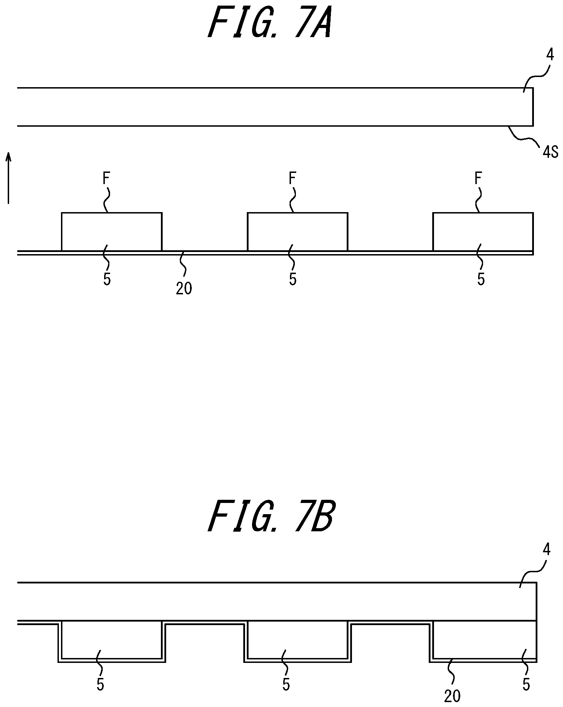

Here, an example of an attachment method of the sole 5 according to this embodiment will be explained using FIGS. 7A and 7B. FIG. 7A is a schematic drawing illustrating the sole 5 and a retaining film 20 before the sole is attached to the ground contact portion 4. FIG. 7A illustrates a state of the ground contact portion 4 and the sole 5 in a side view. As illustrated in FIG. 7A, the land portions constituting the sole 5 are held by the retaining film 20 in a state before the sole 5 is attached to the ground contact portion 4. The land portions constituting the sole 5 are, for example, attached to the retaining film 20. An adhesive is applied to an attachment surface F of the sole 5.

FIG. 7B is a schematic drawing illustrating a state that the sole 5 illustrated in FIG. 7A is adhered to the ground contact portion 4. FIG. 7B also illustrates a state of the ground contact portion 4 and the sole 5 in a side view. As indicated by an arrow of FIG. 7A, the sole 5 is attached to the ground contact portion 4 by the adhesive. At this time, the sole 5 and the retaining film 20 which holds the sole 5 may be collectively attached to the ground contact portion 4. In other words, as illustrated in FIG. 7B, also the retaining film 20 may be attached to the ground contact portion 4 together with the sole 5. The retaining film 20 may be constituted by a material having stretch properties such that it can be closely attached to the ground contact portion 4 in a state of covering surfaces other than the attachment surfaces F of the sole 5 as illustrated in FIG. 7B. Also, in a case where vacuuming of the sole 5 and the ground contact portion 4 is executed when the sole 5 is attached to the ground contact portion 4, the retaining film 20 may be constituted by a material which is airtight. As illustrated in FIG. 7B, in a case where the retaining film 20 is attached to the ground contact portion 4 together with the sole 5, when the wearer starts to use the athletic prosthetic leg 1 with the sole 5 attached, the retaining film 20 contacts the road surface S, not the land portions of the sole 5. In this case, the retaining film 20 is preferably constituted by a material which is easily worn. Since the retaining film 20 is constituted by the material which is easily worn, after the start of use of the athletic prosthetic leg 1 by the wearer, the retaining film 20 is worn more rapidly. This achieves contact of the land portions of the sole 5 and the road surface S which has been originally intended. Additionally, it should be understood that the retaining film 20 explained with reference to FIGS. 7A and 7B is used for attachment of the sole 5, and does not constitute a part of the sole 5 according to the second embodiment.

Additionally, in the above explanation, it has been explained that the adhesive is applied to the attachment surface F of the sole 5 and the sole 5 is attached by the adhesive. However, the attachment is not necessarily executed by the adhesive. For example, in a case where the sole 5 is constituted by a material to be melted by heat, in a state that the sole 5 and the retaining film 20 collectively contacts the ground contact portion 4, heat is applied to the sole 5, thereby attaching the sole 5 to the ground contact portion 4.

Also, in an example illustrated in FIG. 7B, the retaining film 20 is also attached to the ground contact portion 4. However, the retaining film 20 is not necessarily attached to the ground contact portion 4. The retaining film 20 may be peeled off after the sole 5 is attached to the ground contact portion 4.

The attachment method of the sole 5 according to this embodiment is not limited to one described above. For example, the sole 5 may be attached to the ground contact portion 4 by attaching a respectively independent land portion one by one to the ground contact portion 4. In this case, for example, the adhesive may be applied to the ground contact region 4s of the ground contact portion 4.

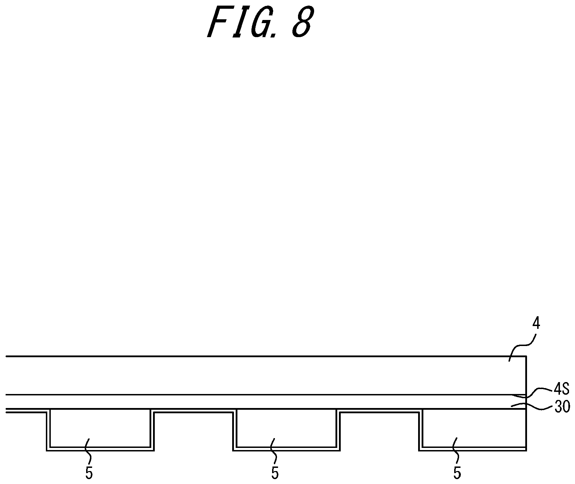

In the first embodiment and the second embodiment described above, a case where the sole 5 is attached to the athletic prosthetic leg 1 such that the sole 5 directly contacts the ground contact portion 4 has been explained. However, the sole 5 is not necessarily attached to the athletic prosthetic leg 1 such that the sole 5 directly contacts the ground contact portion 4. For example, as illustrated in FIG. 8 as a side view, a cushion member 30 may be interposed between the sole 5 and the ground contact region 4s. In this case, in a state that the sole 5 is attached to the ground contact region 4s of the athletic prosthetic leg 1, a surface to which the sole 5 is attached is constituted by the cushion member 30.

Additionally, in any of the embodiments described above, in the pattern of the bottom surface 5s of the sole, fluorine is preferably applied to a groove wall and a groove bottom constituting a width direction groove which defines width direction land portions. Since the fluorine is applied to the groove wall and the groove bottom of the width direction groove, drainage performance in the bottom surface 5s of the sole can be improved.

### REFERENCE SIGNS LIST

- 1: athletic prosthetic leg
- 2: leg portion
- 2a: straight portion
- 2b, 2c: curved portion
- 3: curved portion
- 4: ground contact portion
- 4s: ground contact region
- 5: sole
- 5s: bottom surface
- 6: toe-side tab for sticking
- 7: curved-portion-side tab for sticking
- 9: through hole
- 10, 11, 12, 14: land portion
- 10a, 11a, 12a: width direction extending portion
- 10b, 11b, 12b: toe side protruding portion
- 10c, 11c, 12c: curved portion side protruding portion
- 13: linear groove
- 20: retaining film
- 30: cushion member
- B1, B2: border
- BL: line
- C: point
- F: attachment surface
- L1: maximum length
- M1: center
- Q1: heel side region
- S: road surface
- T: toe
- W: width direction
- X1, X2: arc
- Y: leg portion front-rear direction
- th1, th2, th3: thickness
- w2, w3,: w4 land portion width

## Claims

1. A sole for a prosthetic leg, the sole being configured to be attached to a ground contact region of the prosthetic leg with a road surface, the sole comprising a through hole at least on a partial region.

2. The sole for the prosthetic leg according to claim 1, further comprising a land portion defined by a groove portion at a side of a bottom surface, wherein the sole comprises the through hole on the groove portion.

3. A sole for a prosthetic leg, the sole being configured to be attached to a ground contact region of the prosthetic leg with a road surface, the sole being divided into a plurality of sole portions.

4. The sole for the prosthetic leg according to any one of claims 1 to 3, wherein the sole is attached to the prosthetic leg via a cushion member.
